# NEUE EUROPÄISCHE PATENTSCHRIFT

(11) **EP 2 885 269 B2**
(45) Veröffentlichungstag und Bekanntmachung des Hinweises auf die Entscheidung über den Einspruch: **26.07.2023**
(45) Hinweis auf die Patenterteilung: 14.09.2016
(21) Anmeldenummer: 13735210.0
(22) Anmeldetag: 11.07.2013
(51) Int. Cl.: C07C 209/52, C07C 249/02, C07C 209/26

(54) **KONTINUIERLICHES VERFAHREN ZUR HERSTELLUNG PRIMÄRER ALIPHATISCHER AMINE AUS ALDEHYDEN**
CONTINUOUS METHOD FOR PRODUCING PRIMARY ALIPHATIC AMINES FROM ALDEHYDES
PROCÉDÉ CONTINU POUR PRODUIRE DES AMINES ALIPHATIQUES PRIMAIRES À PARTIR D'ALDÉHYDES

(30) Priorität: 17.08.2012 DE 102012016433
(43) Veröffentlichungstag der Anmeldung: 24.06.2015
(73) Patentinhaber: OQ Chemicals GmbH, 40789 Monheim am Rhein (DE)
(72) Erfinder: BERMANN, Dirk, 45475 Mülheim (DE); EISENACHER, Matthias, 46485 Wesel (DE); GEISEL, Sebastian, 45147 Essen (DE); JOHNEN, Leif, 46286 Dorsten (DE); HEYMANNS, Peter, 45147 Essen (DE); NOWOTNY, Norman, 45276 Essen (DE); SCHALAPSKI, Kurt, 46147 Oberhausen (DE); STRUTZ, Heinz, 47445 Moers (DE)
(74) Vertreter: Michalski Hüttermann & Partner Patentanwälte mbB
(86) Internationale Anmeldenummer: PCT/EP2013/002069
(87) Internationale Veröffentlichungsnummer: WO 2014/026726

(56) Entgegenhaltungen:
- EP-A2- 0 135 106
- EP-A2- 1 106 600
- EP-B1- 1 529 768
- WO-A1-2008/006750
- WO-A1-2011/067199
- WO-A2-03/076386
- DE-A1-102010 045 142
- DE-B4- 19 859 776
- GB-A- 1 421 278
- US-A1- 2005 107 637
- Achim Fisher Et Al: "Continuous Animation of Propanediols in Supercritical Ammonia", Angew. Chem. Int. Ed., vol. 38, no. 3, 1999, pages 351-354,
- Fischer A. et al: "Cobalt-Catalyzed Amination of 1,3-Propanediol: Effects of Catalyst Promotion and Use of Supercritical Ammonia as Solvent and Reactant", Journal of Catalysis, vol. 183, 1999, pages 373-383,
- BAIKER A: "Supercritical Fluids in Heterogeneous Catalysis", Chem. Rev., vol. 99, 1999, pages 453-473,
- Jenzer Gregor: "Cobalt?catalyzed amination of 1,3?cyclohexanediol and 2,4?pentanediol in supercritical ammonia", Catalysis Letters, vol. 61, 1999, pages 111-114,
- Gomez et al, Adv. Synth. Catal., 2002, 344, 1037

## Beschreibung

Die vorliegende Erfindung betrifft ein kontinuierliches Verfahren zur Herstellung von primären aliphatischen Aminen mit 3 bis 18 Kohlenstoffatomen durch reduktive Aminierung des entsprechenden aliphatischen Aldehyds mit Ammoniak in Gegenwart eines Hydrierkatalysators oberhalb des kritischen Punktes von Ammoniak.

Aliphatische Amine sind wichtige organische Zwischenprodukte, die in großem industriellem Maßstab hergestellt werden. Beispielsweise werden sie für die Herstellung von Agrochemikalien oder Farbstoffen weiterverarbeitet oder sie dienen als Zusatz in oberflächenaktiven Formulierungen, als Korrosionsinhibitor in Schmiermitteln oder als Hilfsstoffe für die Papier, Textil und Kautschukindustrie.

Es ist bekannt, primäre aliphatische Amine aus Aldehyden und Ammoniak mit Wasserstoff an einem Katalysator herzustellen. Man bezeichnet diese Reaktion auch als reduktive Aminierung. Die Aminbildung kann durch folgende Reaktionsstufen beschrieben werden:

R-C(=O)H + NH₃ → R-C(=NH)H + H₂O (1)

R-C(=NH)H + H₂ → R-CH₂-NH₂ (2)

In der ersten Reaktionsstufe entsteht zunächst unter Wasserabspaltung ein Imin, das anschließend in einer zweiten Reaktionsstufe katalytisch hydriert wird.

Bei der Reaktionsführung treten jedoch unerwünschte Nebenreaktionen auf. Zum einen kann es durch die Direkthydrierung der Einsatzaldehyde zur Alkoholbildung kommen. Auch kann der Einsatzaldehyd in dem basischen Medium einer Aldolkondensation unterliegen und bereits gebildetes primäres Amin kann mit dem Einsatzaldehyd über die Azomethinzwischenstufe zu einem sekundären Amin abreagieren, das dann noch analog zu einem tertiären Amin weiterreagieren kann. Auch enthalten die Aldolkondensationsprodukte reaktive Gruppen, die mit den stickstoffhaltigen Verbindungen höhersiedende Kondensationsprodukte bilden können. Um die Selektivität in Richtung der primären aliphatischen Amine zu verbessern und um die Bildung von hochsiedenden Nebenprodukten zurückzudrängen, schlägt der Stand der Technik verschiedene Maßnahmen vor, beispielsweise die Verwendung eines Ammoniaküberschusses oder eines Lösungsmittels, wenn damit zu rechnen ist, dass das Reaktionsgemisch durch das gebildete Wasser inhomogen wird (Houben-Weyl, Methoden der organischen Chemie, 4. Auflage, Georg Thieme Verlag Stuttgart, Bd XI/1, Seite 602 ff.).

DE 936211 beschreibt einen Flüssigphasenprozess zur Herstellung primärer aliphatischer Amine. Dabei wird der umzusetzende Aldehyd zunächst mit Ammoniak bei Temperaturen unterhalb von 0°C vermischt. Gegebenenfalls werden die Aldehyde mit einem niedrig siedenden Alkohol, beispielsweise Methanol, verdünnt. Anschließend wird diese Mischung bei erhöhter Temperatur und erhöhtem Druck in der Sumpf- oder Rieselfahrweise katalytisch hydriert, beispielsweise an einem Kobalt- oder Nickelkontakt.

Nach DE 199 35 448 A1 wird eine Mischung aus Methanol und Ammoniak mit Raney-Nickel versetzt und nach Beaufschlagung mit Wasserstoff auf Reaktionstemperatur aufgeheizt. Anschließend wird der Aldehyd zudosiert. Nach-Beendigung der Reaktion wird der Ansatz entspannt, wobei Methanol und Ammoniak verdampfen. Anschließend wird das zurückgebliebene primäre aliphatische Amin weiter umgesetzt.

Aus DE 10 2010 045 142 A1 ist ein lösungsmittelfreies Verfahren in der Flüssigphase bekannt, bei dem der Einsatzaldehyd mit Wasserstoff und Ammoniak in Gegenwart eines Hydrierkatalysators bei einer Temperatur von 100 bis 170°C und bei einem Druck von 6 bis 11 MPa zur Reaktion gebracht wird und wobei je Mol Einsatzaldehyd mindestens 30 Mol Ammoniak eingesetzt werden.

Der Stand der Technik weist ebenfalls daraufhin, in dem reduktiven Aminierungsprozess Ammoniak bei überkritischen Bedingungen einzusetzen. Die kritische Temperatur von Ammoniak liegt bei 132,5°C und der kritische Druck bei 112,5 atm (Handbook of Chemistry and Physics, 50th Edition 1969, The Chemical Rubben CO, Seite F-64), entsprechend 11,4 MPa. Nach GB 1,421,278 wird die Reaktionstemperatur bis auf 160°C und der Überdruck bis auf 125 atm (entsprechend 12,7 MPa) begrenzt. In einer bevorzugten Ausführungsform wird das molare Verhältnis von Ammoniak zu Einsatzaldehyd bis auf 16 zu 1 eingeschränkt. Das Ausführungsbeispiel 4 von GB 1,421,278 offenbart die reduktive Aminierung von Isobutyraldehyd mit Ammoniak bei einer Reaktionstemperatur von 140°C und bei einem Überdruck von 125 atm (12,7 MPa Überdruck).

Gemäß WO97/38955 A1 wird die reduktive Aminierung von Aldehyden in einem kontinuierlichen Verfahren in Gegenwart eines heterogenen Katalysators durchgeführt, wobei sich wenigstens ein Reaktionspartner, zusätzlich zu Wasserstoff, im überkritischen Zustand oder nahe am überkritischen Zustand befindet. Vorzugsweise erfolgt die Umsetzung in Gegenwart eines Lösungsmittels, das unter den Reaktionsbedingungen selbst im überkritischen Zustand vorliegt, so dass Wasserstoff und die übrigen Reaktionskomponenten in einer homogenen Phase anwesend sind.

US 2005/0107637 A1 behandelt die reduktive Aminierung von hydroformyliertem Polyisobuten mit Ammoniak in Gegenwart eines heterogenen Katalysators bei erhöhter Temperatur und bei überkritischem Druck.

Aus dem Stand der Technik kann nicht generell hergeleitet werden, dass aliphatische Aldehyde mit einer mittleren Kohlenstoffzahl bei Bedingungen oberhalb der kritischen Bedingungen für Ammoniak mit hoher Selektivität in primäre aliphatische Amine überführt werden. Häufig wird die Bildung von hochsiedenden Nebenprodukten beobachtet, die die Selektivität und damit die Ausbeute an dem gewünschten primären aliphatischen Amin mindern. Darüber hinaus neigen die Verfahren entsprechend dem Stand der Technik zu einer nur begrenzten Katalysatorstandzeit, wodurch neben der angesprochenen verminderten Ausbeute an dem gewünschten Amin auch wirtschaftliche Nachteile für den Aminierungsprozess verbunden sind.

Aufgabe der vorliegenden Erfindung ist es daher, ein Verfahren zur Herstellung von primären aliphatischen Aminen bereitzustellen, das einen geringen technischen Aufwand benötigt und mit dem die gewünschten primären aliphatischen Amine mit hoher Selektivität erhalten werden. Insbesondere soll die Bildung von hochsiedenden Nebenprodukten möglichst zurückgedrängt werden. Gleichzeitig soll auch die Standzeit des Hydrierkatalysators verlängert werden.

Gegenstand der vorliegenden Erfindung ist daher ein kontinuierliches Verfahren zur Herstellung von primären aliphatischen Aminen mit 3 bis 18 Kohlenstoffatomen durch Umsetzung von entsprechenden aliphatischen Aldehyden mit Ammoniak und Wasserstoff in Gegenwart eines Hydrierkatalysators. Das Verfahren ist dadurch gekennzeichnet, dass die Umsetzung lösungsmittelfrei bei einem molaren Verhältnis von aliphatischem Aldehyd zu Ammoniak von mehr als 1 zu 16 oberhalb der kritischen Temperatur und oberhalb des kritischen Drucks von Ammoniak durchgeführt wird.

Überraschender Weise kann bei der lösungsmittelfreien, in Bezug auf Ammoniak überkritischen Verfahrensführung die Bildung von selektivitätsmindernden Hochsiedern auf unter 10 Gew.-% im Reaktionsprodukt gedrückt werden, wenn bei der kontinuierlichen Prozessführung die Reaktionsbedingungen gezielt eingestellt werden. Ein weiterer, nicht zu erwartender Vorteil des erfindungsgemäßen Verfahrens liegt bei der Verlängerung der Katalysatorstandzeit, wenn Ammoniak oberhalb seines kritischen Punktes umgesetzt wird. Auch ein separates Vormischen der Einsatzprodukte Ammoniak und aliphatischer Aldehyd unter Kühlung ist nicht erforderlich. Die Einsatzprodukte werden ohne weitere apparative Maßnahmen direkt aus ihren Vorlagen getrennt aber gleichzeitig in den Aminierungsreaktor gegeben.

Unter dem Begriff lösungsmittelfrei im Sinne der vorliegenden Erfindung versteht man, auf den aktiven Zusatz eines Lösungs- oder Verdünnungsmittels zu verzichten. Hingegen können geringe Mengen von Nebenbestandteilen in den Einsatzprodukten, beispielsweise herstellungsbedingte Alkoholreste in den Einsatzaldehyden, die Lösungsmittel- oder Verdünnungsmitteleigenschaften aufweisen, zugegen sein. Auch das während der Reaktion gebildete Wasser fällt nicht unter den Begriff Lösungsmittel oder Verdünnungsmittel, obwohl es gegenüber Ammoniak als Lösungsmittel wirkt.

Je Mol aliphatischen Aldehyd werden mehr als 16 Mol Ammoniak, vorzugsweise wenigstens 18 Mol Ammoniak und insbesondere wenigstens 20 Mol Ammoniak eingesetzt. Obwohl der hohe Ammoniaküberschuss zu einem Verdünnungseffekt führt und so der Bildung von Hochsiedern entgegenwirkt, sollte dennoch aufgrund des hohen Anteils der basisch reagierenden Verbindung die Aldehydkondensation in erheblichem Maße ablaufen, zumal nach der erfindungsgemäßen Arbeitsweise kein zugesetztes Lösungs- oder Verdünnungsmittel zugegen ist. Überraschenderweise kann trotz eines hohen Ammoniaküberschusses die Bildung von hochsiedenden Kondensationsprodukten zurückgedrängt werden, wenn die reduktive Aminierung oberhalb der kritischen Temperatur und oberhalb des kritischen Drucks für Ammoniak durchgeführt wird. Für das Erreichen wirtschaftlich vertretbarer Ausbeuten an primären aliphatischen Aminen ist es wesentlich, je Mol aliphatischen Aldehyd mehr als 16 Mol Ammoniak einzusetzen. Eine Obergrenze über den Ammoniaküberschuss ist nicht kritisch und wird nur durch verfahrenstechnische oder wirtschaftliche Überlegungen festgelegt. In einer bevorzugten Ausführungsform der Erfindung beträgt das Molverhältnis von aliphatischem Aldehyd zu Ammoniak von 1 zu 18 bis 1 zu 60, vorzugsweise von 1 zu 20 bis 1 zu 50.

Die Umsetzung des aliphatischen Aldehyds mit Ammoniak in Gegenwart von Wasserstoff an dem Hydrierkatalysator erfolgt oberhalb der kritischen Bedingungen von Ammoniak, insbesondere bei Temperaturen oberhalb von 132,5°C bis 190°C, bevorzugt oberhalb von 132,5°C bis 180°C und ganz besonders bevorzugt bei Temperaturen oberhalb von 160°C bis 180°C. Der Reaktionsdruck, der Gesamtdruck, wird vorzugsweise oberhalb von 11,4 MPa, dem kritischen Druck von Ammoniak, bis auf 20 MPa, eingestellt, insbesondere oberhalb von 12,8 bis 20 MPa. Ganz besonders wird ein Reaktionsdruck von 13 bis 18 MPa eingestellt.

Als Hydrierkatalysatoren dienen übliche, bei der reduktiven Aminierung von Carbonylverbindungen verwendete Katalysatoren, die mindestens ein Metall der Nebengruppe 8 bis 11 des Periodensystems der Elemente enthalten, wie Nickel, Kobalt, Platin, Palladium, Eisen, Rhodium oder Kupfer. Besonders bevorzugt sind Nickel- oder Kobaltkatalysatoren. Neben trägerfreien Katalysatoren, wie Raney-Nickel oder Raney-Kobalt, können auch geträgerte Katalysatoren eingesetzt werden. Bei geträgerten Katalysatoren liegt im Allgemeinen das katalytisch aktive Metall in einer Menge von etwa 5 bis 70 Gew.-%, vorzugsweise etwa 10 bis etwa 65 Gew.-% und insbesondere etwa 20 bis 60 Gew.-%, jeweils bezogen auf das Gesamtgewicht des Hydrierkatalysators vor. Als Katalysatorträger eignen sich alle herkömmlichen Trägermaterialien, zum Beispiel Aluminiumoxid, Aluminiumoxidhydrate in ihren verschiedenen Erscheinungsformen, Siliciumdioxid, Polykieselsäuren (Kieselgele) einschließlich Kieselgur, Kieselxerogele, Magnesiumoxid, Zinkoxid, Zirconiumoxid und Aktivkohle. Neben den Hauptkomponenten katalytisch aktives Metall und Trägermaterial können die Hydrierkatalysatoren noch Zusatzstoffe in untergeordneten Mengen enthalten, die zum Beispiel der Verbesserung ihrer Hydrieraktivität und/oder ihrer Standzeit und/oder ihrer Selektivität dienen. Derartige Zusatzstoffe sind bekannt, zu ihnen gehören zum Beispiel die Oxide des Calciums, Bariums, Zinks, Aluminiums, Zirconiums und Chroms. Sie werden dem Hydrierkatalysator im Allgemeinen in einem Anteil von insgesamt 0,1 bis 30 Gew.-%, bezogen auf das Gesamtgewicht des Hydrierkatalysators, zugesetzt. Als bevorzugtes katalytisch aktives Metall hat sich Nickel erwiesen. Insbesondere sind Nickelkatalysatoren auf Kieselgur als Trägermaterial mit Chrom als Zusatzstoff für das erfindungsgemäße Aminierungsverfahren geeignet. Ganz besonders geeignet sind Nickelkatalysatoren, die 20 bis 60 Gew.-% Nickel, von 20 bis 70 Gew.-% Kieselgur und von 10 bis 20 Gew.-% Chrom enthalten, jeweils bezogen auf das Gesamtgewicht des Hydrierkatalysators und gegebenenfalls Füllstoffe mit Rest auf 100 Gew.-%.

Die reduktive Aminierung wird beispielsweise an fest angeordneten Katalysatoren nach der Rieselfahrweise oder Sumpffahrweise sowie im Wirbelschichtverfahren durchgeführt. Neben Wasserstoff werden aliphatischer Aldehyd und Ammoniak dem Aminierungsreaktor getrennt aber gleichzeitig aus ihren Vorlagen zugeführt.

Vorzugsweise wird die kontinuierliche reduktive Aminierung in einem Rohrreaktor an fest angeordneten Hydrierkatalysatoren durchgeführt. Unter einem Rohrreaktor ist auch ein Bündel von mehreren eng parallel geschalteten Rohren zu verstehen. Die eingesetzten Rohrreaktoren können ebenfalls Füllkörper oder Einbauten enthalten, beispielsweise, Raschigringe, Sättel, Pallringe, Filterplatten oder Kolonnenböden, sowie gegebenenfalls Rührvorrichtungen. In einer besonders bevorzugten Ausgestaltung erfolgt die reduktive Aminierung in einem Rohrreaktor jedoch ohne Einbauten mit geschüttetem Hydrierkatalysator.

Bei der kontinuierlichen Fahrweise hat sich eine Katalysatorbelastung V/Vh mit aliphatischem Aldehyd, ausgedrückt in Durchsatzvolumen an aliphatischem Aldehyd pro Katalysatorvolumen und Zeit, von 0,02 - 1,00 h⁻¹, vorzugsweise 0,10 - 0,80 h⁻¹, als zweckmäßig erwiesen. Eine höhere Belastung des Hydrierkatalysators mit aliphatischem Aldehyd ist zu vermeiden, weil dann die reduktive Aminierung nicht mehr vollständig erfolgt und aufgrund des hohen Aldehydrestgehaltes eine verstärkte Bildung von hochsiedenden Nebenprodukten beobachtet wird.

Bei zu geringen Durchsätzen pro Zeiteinheit wird die Anlagenkapazität nicht optimal ausgenutzt.

Die reduktive Aminierung erfolgt neben Ammoniak als Ausgangsverbindung vorzugsweise mit reinem Wasserstoff. Neben reinem Wasserstoff können jedoch auch Gemische verwendet werden, die freien Wasserstoff und daneben unter den Bedingungen der reduktiven Aminierung inerte Bestandteile enthalten.

Die nach dem erfindungsgemäßen Verfahren umzusetzenden aliphatischen Aldehyde enthalten 3 bis 18 Kohlenstoffatome im Molekül, vorzugsweise 5 bis 15 und ganz bevorzugt 8 bis 15. Die Herkunft der aliphatischen Aldehyde ist nicht auf bestimme Herstellungsverfahren beschränkt.

Aufgrund ihrer leichten Zugänglichkeit werden durch Oxo-Synthese oder Hydroformylierung, d.h. durch Reaktion der entsprechenden um ein Kohlenstoffatom verminderten Olefine mit Kohlenmonoxid und Wasserstoff gewonnene Aldehyde bevorzugt, wobei sowohl geradkettige als auch verzweigte Olefine sowie alicyclische Olefine, beispielsweise Dicyclopentadien oder Olefinoligomere wie Tripropylen oder Tetrapropylen, als Ausgangsmaterialien für die Hydroformylierungsreaktion verwendet werden können. Als aliphatische Aldehyde eignen sich sowohl geradkettige n- als auch verzweigtkettige iso-Aldehyde sowie alicyclische Aldehyde entweder in reiner Form oder als Gemisch mit isomeren Aldehyden der gleichen Kohlenstoffzahl. Auch Gemische von aliphatischen Aldehyden mit unterschiedlicher Kohlenstoffzahl können verwendet werden. Besonders lassen sich nach der erfindungsgemäßen Arbeitsweise aliphatische Aldehyde mit 8 bis 15 Kohlenstoffatomen in die entsprechenden primären aliphatischen Amine überführen, beispielsweise 2-Ethylhexanal, Octanal, Nonanal, Decanal, Undecanal, Dodecanal, Tridecanal, Tetradecanal oder Pentadecanal oder Mischungen davon. Die entsprechenden Aldehyde können dabei als geradkettige Verbindungen, als verzweigte Strukturisomere oder im Gemisch aus geradkettigen und verzweigten Strukturisomeren, auch mit unterschiedlicher Kohlenstoffzahl, eingesetzt werden. Das Gemisch aus geradkettigen n- und verzweigtkettigen iso-Aldehyden, die 13 und 15 Kohlenstoffatome im Molekül enthalten, ist für das erfindungsgemäße Aminierungsverfahren ganz besonders geeignet.

Das dem Aminierungsreaktor entnommene Reaktionsgemisch wird in einen Hochdruckabscheider geführt, wobei es zur Bildung einer gasförmigen und flüssigen Phase kommt. Die gasförmige Phase enthält im Wesentlichen Ammoniak und Wasserstoff sowie geringe Mengen an Reaktionswasser und wird abgeführt. Die erhaltene flüssige Phase wird über eine Standregelung auf Normaldruck entspannt und fließt in einen Vorlagebehälter. Bei dem Entspannungsvorgang entgast in der Flüssigphase gelöstes Ammoniak und gelöster Wasserstoff, die als Entspannungsabgas aus dem Vorlagebehälter abgeführt werden. Aus dem abgeführten Abgas des Hochdruckabscheiders und aus dem Entspannungsabgas kann Ammoniak zurückgewonnen und wieder in den reduktiven Aminierungsprozess zurückgefahren werden.

Aus der im Vorlagebehälter angesammelten flüssigen Phase werden anschließend Restmengen an Ammoniak und das Reaktionswasser entfernt. Das erhaltene primäre aliphatische Amin wird anschließend nach an sich bekannten Verfahren, beispielsweise durch Destillation, zu spezifikationsgerechter Ware aufgereinigt.

Den während des kontinuierlichen Betriebs gelegentlich beobachteten Katalysatoralterungseffekten, die sich in der Regel in einer Zunahme des Hochsiederanteils äußern, kann durch eine Erhöhung der Reaktionstemperatur während des laufenden Prozesses, beispielsweise ausgehend oberhalb von 132,5°C bis auf 190°C entgegengewirkt werden. Dadurch kann die Standzeit des Hydrierkatalysators verlängert werden.

Durch das erfindungsgemäße Verfahren lassen sich aliphatische Aldehyde bei hohem Umsatz mit hoher Selektivität in die entsprechenden primären aliphatischen Amine überführen. Der Gehalt an hochsiedenden Nebenprodukten, gaschromatographisch ermittelt, in dem nach Abtrennung von Ammoniak und gebildetem Reaktionswasser erhaltenen Rohprodukt liegt dabei unter 10%.

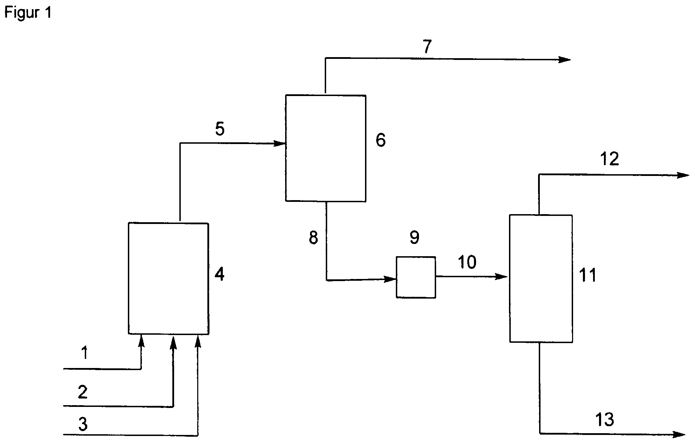

Das erfindungsgemäße Verfahren wird im Folgenden anhand des Prinzipschemas gemäß Figur 1 näher erläutert. Das erfindungsgemäße Verfahren ist aber nicht auf die in der Zeichnung dargestellte Ausführungsform beschränkt.

Über die Leitung (1) wird Ammoniak, über die Leitung (2) wird Wasserstoff und über die Leitung (3) aliphatischer Aldehyd in den mit dem Hydrierkatalysator gefüllten Aminierungsreaktor (4) kontinuierlich eingeleitet, der oberhalb der kritischen Bedingungen von Ammoniak betrieben wird. Der Reaktoraustrag wird über die Leitung (5) abgezogen und in einen Hochdruckabscheider (6) geführt, in dem es zur Bildung einer gasförmigen und flüssigen Phase kommt. Die Gasphase aus dem Hochdruckabscheider (6) wird über die Leitung (7) abgeführt. Aus der abgeführten Gasphase, die im Wesentlichen aus Ammoniak und Wasserstoff besteht und Reaktionswasser in geringen Mengen enthält, wird Ammoniak zurückgewonnen und wieder über Leitung (1) in den Prozess zurückgeführt (nicht in Figur 1 gezeigt). Die im Hochdruckabscheider (6) erhaltene Flüssigphase wird über die Leitung (8) abgeführt, über eine Standregelung (9) auf Normaldruck entspannt und über Leitung (10) drucklos in den Vorlagebehälter (11) geführt. Die beim Entspannungsvorgang gebildeten gasförmigen Anteile, im Wesentlichen Restmengen aus gelöstem Ammoniak und Wasserstoff werden über die Leitung (12) ausgeschleust. Gegebenenfalls kann Ammoniak aus dem abgeführten Strom zurückgewonnen und zusammen mit frischem Ammoniak über Leitung (1) dem Aminierungsreaktor (4) wieder zugeführt werden (nicht in Figur 1 gezeigt). Über Leitung (13) wird die entgaste flüssige Phase abgeführt und anschließend nach an sich bekannten Verfahren destillativ aufgearbeitet (nicht in Figur 1 gezeigt).

Im Folgenden wird das erfindungsgemäße Verfahren anhand einiger Beispiele näher erläutert.

### Versuchsaufbau

Die reduktive Aminierung erfolgte an einem kommerziellen, kieselgurgeträgerten Nickelkatalysator mit Chrom als Zusatzstoff in einem Rohrreaktor in der Sumpffahrweise. Als aliphatischer Aldehyd kam ein Gemisch aus geradkettigen n- und verzweigtkettigen iso-C₁₃- und C₁₅-Aldehyden zum Einsatz. Einsatzaldehyd, Ammoniak und Wasserstoff wurden getrennt aber gleichzeitig am Sumpf des Rohrreaktors kontinuierlich zugeführt. Über den Kopf des Rohrreaktors entnahm man das Reaktionsprodukt und leitete es in einen Hochdruckabscheider. Die anfallende Flüssigkeit wurde über eine Standregelung auf Normaldruck entspannt und in eine drucklose Vorlage geführt. Das erhaltene organische Rohprodukt wurde anschließend gaschromatographisch analysiert.

Die Reaktionsbedingungen und die kontinuierliche Zufuhr der Einsatzstoffe wurden gemäß den Bedingungen den nachfolgenden Tabellen 1, 2 und 3 eingestellt.

In den Tabellen ist ebenfalls die gaschromatographisch ermittelte Zusammensetzung des organischen Produkts, ammoniak- und wasserfrei, angegeben (in Fl.- %).

Das für die Versuche in den Tabellen 1-3 verwendete aliphatische Aldehydgemisch wies folgende typische Zusammensetzung auf (gaschromatographisch ermittelt, Angaben in FI.-Prozent):

| | |
|---|---|
| C₁₂ und C₁₄-KW | 2,1 |
| n-/I-C₁₃-Aldehyd | 62,5 |
| n-/i-C₁₅-Aldehyd | 34,9 |
| Nachlauf C26-C30 | 0,5 |

**Tabelle 1: Reduktive Aminierung eines n-/iSO-C₁₃/C₁₅-Aldehydgemischs und gaschromatographische Analysen des erhaltenen rohen primären aliphatischen Amins in FI.-% (ammoniak- und wasserfrei)**

| | **Beispiel 1** (Vergleich; Beispiel 4 aus GB 1,421,278) | **Beispiel 2** (Vergleich; Angaben aus Anspruch 4 von GB 1,421,278) | **Beispiel 3** | **Beispiel 4** |
|---|---|---|---|---|
| Katalysatormenge [ml] | 1950 | 1950 | 1950 | 1950 |
| Druck [MPa], gesamt | 12,7 | 12,7 | 13,0 | 13,0 |
| Katalysatortemperatur [°C] | 140 | 140 | 140 | 140 |
| Einsatz Wasserstoff [g/h] | 30 | 30 | 31 | 30 |
| Einsatz Aldehyd [g/h] | 402 | 402 | 401 | 400 |
| Einsatz Ammoniak [g/h] | 130 | 520 | 800 | 1500 |
| VlVh bezogen auf Aldehyd [1/h] | 0,25 | 0,25 | 0,25 | 0,25 |
| Molares Verhältnis Aldehyd/Ammoniak | 1:4 | 1:16 | 1:25 | 1:47 |
| **Produktanalyse** | | | | |
| Vorlauf | 0,1 | 2,9 | 0,6 | 0,2 |
| C₁₂ und C₁₄-KW | 2,1 | 2,4 | 1,8 | 1,9 |
| Alkohole | 0,1 | 0,5 | 0,4 | 0,7 |
| Nitrile | 0,1 | 0,3 | 0,1 | 0,1 |
| n/i-C₁₃-Amin | 2,3 | 53,6 | 62,7 | 62,1 |
| n/i-C₁₅-Amin | 1,2 | 24,4 | 31,7 | 33,1 |
| Höhersieder | 94,1 | 15,9 | 2,7 | 1,9 |
| **Summe** | 100,00 | 100,00 | 100,00 | 100,00 |

Die Vergleichsbeispiele 1 und 2 zeigen die Ergebnisse der reduktiven Aminierung eines n-/iso-C₁₃/C₁₅-Aldehydgemischs unter den Bedingungen, die in GB1,421,278 als geeignet beschrieben werden. Auffällig ist die massive Hochsiederbildung, wenn unter den aus dem Stand der Technik bekannten Bedingungen gearbeitet wird. Wenn hingegen nach den erfindungsgemäßen Beispielen 3 und 4 das molare Ammoniak zu Aldehydverhältnis über das in GB 1,421,278 empfohlene Verhältnis erhöht wird, kann die Hochsiederbildung in einem unerwartet deutlichem Maße verringert werden.

**Tabelle 2: Reduktive Aminierung eines n-/iso-C₁₃/C₁₅-Aldehydgemischs und gaschromatographische Analysen des erhaltenen rohen primären aliphatischen Amins in Fl.-% (ammoniak- und wasserfrei)**

| | **Beispiel 5** (Vergleich) | **Beispiel 6** (Vergleich) | **Beispiel 7** | **Beispiel 8** | **Beispiel 9** | **Beispiel 10** | **Beispiel 11** |
|---|---|---|---|---|---|---|---|
| Katalysatormenge [ml] | 1950 | 1950 | 1950 | 1950 | 1950 | 1950 | 1950 |
| Druck [MPa], gesamt | 12,7 | 12,7 | 13,0 | 13,0 | 13,0 | 13,0 | 16,0 |
| Katalysatortemperatur [°C] | 140 | 160 | 140 | 150 | 170 | 180 | 180 |
| Einsatz Wasserstoff [g/h] | 30 | 30 | 30 | 29 | 30 | 30 | 30 |
| Einsatz Aldehyd [g/h] | 900 | 902 | 900 | 900 | 901 | 900 | 900 |
| Einsatz Ammoniak [g/h] | 1140 | 1140 | 1500 | 1500 | 1500 | 1500 | 1500 |
| V/Vh bezogen auf Aldehyd [1/h] | 0,56 | 0,56 | 0,56 | 0,56 | 0,56 | 0,56 | 0,56 |
| Molares Verhältnis Aldehyd/Ammoniak | 1:16 | 1:16 | 1:21 | 1:21 | 1:21 | 1:21 | 1:21 |
| **Produktanalyse** | | | | | | | |
| Vorlauf | 0,0 | 0,0 | 0,0 | 0,1 | 0,0 | 0,0 | 0,0 |
| C₁₂ und C₁₄-KW | 1,7 | 1,9 | 1,8 | 1,8 | 1,9 | 2,0 | 2,0 |
| Alkohole | 0,2 | 0,2 | 0,2 | 0,1 | 0,2 | 0,2 | 0,3 |
| Nitrile | 0,1 | 0,1 | 0,1 | 0,1 | 0,1 | 0,1 | 0,1 |
| n/i-C₁₃-Amin | 3,1 | 45,4 | 54,6 | 62,0 | 62,7 | 62,4 | 62,9 |
| n/i-C₁₅-Amin | 0,5 | 21,2 | 26,1 | 32,9 | 33,5 | 33,9 | 33,6 |
| Höhersieder | 94,4 | 31,2 | 17,2 | 3,0 | 1,6 | 1,4 | 1,1 |
| **Summe** | 100,00 | 100,00 | 100,00 | 100,00 | 100,00 | 100,00 | 100,00 |

Vergleichsbeispiel 5 wurde nach den Bedingungen des Vergleichsbeispiels 1 durchgeführt, mit der Ausnahme, dass die Katalysatorbelastung auf 0,56 h⁻¹ angehoben wurde, ein Wert, der ebenfalls in GB 1,421,278 bereichsmäßig erfasst wird. Gegenüber Vergleichsbeispiel 5 wurde in Vergleichsbeispiel 6 die Reaktionstemperatur auf die in Anspruch 1 von GB 1,421,278 genannte Obergrenze angehoben. Bei beiden, im Stand der Technik empfohlenen Reaktionseinstellungen beobachtet man eine deutliche, wirtschaftlich nicht vertretbare Hochsiederbildung.

Wird nach den erfindungsgemäßen Reaktionseinstellungen 7-11 mit einem höheren molaren Verhältnis von Ammoniak zu Aldehyd gearbeitet, kann die Hochsiederbildung reduziert werden, und zwar umso deutlicher, je höher die Reaktionstemperatur ist (Beispiele 7-10). Wird gemäß Beispiel 11 der Druck gegenüber Beispiel 10 von 13 auf 16 MPa erhöht, kann die Hochsiederbildung nochmals vermindert werden.

**Tabelle 3: Reduktive Aminierung eines n-/iso-C₁₃/C₁₅-Aldehydgemischs und gaschromatographische Analysen des erhaltenen rohen primären aliphatischen Amins in FI.-% (ammoniak- und wasserfrei)**

| | **Beispiel 7** (aus Tabelle 2) | **Beispiel 12** | **Beispiel 13** | **Beispiel 14** |
|---|---|---|---|---|
| Katalysatormenge [ml] | 1950 | 1950 | 1950 | 1950 |
| Laufzeit [h] | 212 | 1708 | 1751 | 1847 |
| Druck [MPa], gesamt | 13,0 | 13,0 | 13,0 | 13,0 |
| Katalysatortemperatur [°C] | 140 | 140 | 170 | 180 |
| Einsatz Wasserstoff [g/h] | 30 | 30 | 30 | 30 |
| Einsatz Aldehyd [g/h] | 900 | 900 | 901 | 900 |
| Einsatz Ammoniak [g/h] | 1500 | 1500 | 1500 | 1500 |
| V/Vh bezogen auf Aldehyd [1/h] | 0,56 | 0,56 | 0,56 | 0,56 |
| Molares Verhältnis Aldehyd/Ammoniak | 1:21 | 1:21 | 1:21 | 1:21 |
| **Produktanalyse** | | | | |
| Vorlauf | 0,0 | 0,0 | 0,0 | 0,0 |
| C₁₂ und C₁₄-KW | 1,8 | 2,0 | 1,9 | 2,1 |
| Alkohole | 0,2 | 0,2 | 0,2 | 0,2 |
| Nitrile | 0,1 | 0,1 | 0,1 | 0,1 |
| n/i-C₁₃-Amin | 54,6 | 34,6 | 62,1 | 62,6 |
| n/i-C₁₅-Amin | 26,1 | 15,0 | 33,6 | 33,2 |
| Höhersieder | 17,2 | 48,1 | 2,1 | 1,8 |
| **Summe** | 100,00 | 100,00 | 100,00 | 100,00 |

Die erfindungsgemäßen Bedingungen sind ferner dazu geeignet, Alterungseffekte des Festbett-Katalysators zu kompensieren. Beispiel 7 zeigt das Verhalten des Hydrierkatalysators nach einer Laufzeit von 212 h. Nach einer Laufzeit von insgesamt 1708 h (Beispiel 12) beobachtet man eine deutliche Zunahme an Hochsiedern aufgrund des Alterns des Katalysators. Wie in Beispielen 13 und 14 dargestellt, kann dem Alterungseffekt des Hydrierkatalysators entgegengesteuert werden, wenn die Reaktionstemperatur auf einen Bereich erhöht wird, der außerhalb des in GB 1,421,278 empfohlenen Temperaturbereiches liegt. Durch Temperaturerhöhung über die Laufzeit kann der Alterungsseffekt des Katalysators kompensiert werden und auf diese Weise die Hochsiederbildung vermindert und die Herstellung von primären C_{13/15}-Aminen nachhaltig wirtschaftlich betrieben werden.

## Patentansprüche

1. Kontinuierliches Verfahren zur Herstellung von primären aliphatischen Aminen mit 3 bis 18 Kohlenstoffatomen durch Umsetzung von entsprechenden aliphatischen Aldehyden mit Ammoniak und Wasserstoff in Gegenwart eines Hydrierkatalysators, **dadurch gekennzeichnet, dass** die Umsetzung lösungsmittelfrei bei einem molaren Verhältnis von aliphatischem Aldehyd zu Ammoniak von mehr als 1 zu 16 oberhalb der kritischen Temperatur und oberhalb des kritischen Drucks von Ammoniak durchgeführt wird.

2. Verfahren gemäß Anspruch 1, **dadurch gekennzeichnet, dass** das molare Verhältnis von aliphatischem Aldehyd zu Ammoniak von 1 zu wenigstens 18, vorzugsweise von 1 zu wenigstens 20 beträgt.

3. Verfahren gemäß Anspruch 1 oder 2, **dadurch gekennzeichnet, dass** das molare Verhältnis von aliphatischem Aldehyd zu Ammoniak von 1 zu 18 bis 1 zu 60, insbesondere von 1 zu 20 bis 1 zu 50, beträgt.

4. Verfahren gemäß einem oder mehreren der Ansprüche 1 bis 3, **dadurch gekennzeichnet, dass** die Umsetzung bei einer Temperatur oberhalb von 132,5°C bis 190°C, insbesondere oberhalb von 132,5°C bis 180°C und ganz besonders bevorzugt oberhalb von 160°C bis 180°C erfolgt.

5. Verfahren gemäß einem oder mehreren der Ansprüche 1 bis 4, **dadurch gekennzeichnet, dass** die Umsetzung bei einem Druck oberhalb von 11,4 bis 20 MPa, insbesondere oberhalb von 12,8 bis 20 MPa und ganz besonders bevorzugt von 13 bis 18 MPa erfolgt.

6. Verfahren gemäß einem oder mehreren der Ansprüche 1 bis 5, **dadurch gekennzeichnet, dass** die umzusetzenden aliphatischen Aldehyde 5 bis 15, vorzugsweise 8 bis 15, Kohlenstoffatome im Molekül enthalten.

7. Verfahren gemäß einem oder mehreren der Ansprüche 1 bis 6, **dadurch gekennzeichnet, dass** der Hydrierkatalysator mindestens Nickel, Kobalt, Platin, Palladium, Eisen, Rhodium oder Kupfer enthält.

8. Verfahren gemäß einem oder mehreren der Ansprüche 1 bis 7, **dadurch gekennzeichnet, dass** der Hydrierkatalysator ein Trägermaterial enthält.

9. Verfahren gemäß einem oder mehreren der Ansprüche 1 bis 8, **dadurch gekennzeichnet, dass** der Hydrierkatalysator als Zusatzstoffe Oxide des Calciums, Bariums, Zinks, Aluminiums, Zirconiums, Chroms oder Mischungen davon enthält.

10. Verfahren gemäß einem oder mehreren der Ansprüche 1 bis 9, **dadurch gekennzeichnet, dass** man als Hydrierkatalysator einen Nickelkatalysator verwendet, der 20 bis 60 Gew.-% Nickel, von 20 bis 70 Gew.-% Kieselgur und von 10 bis 20 Gew.-% Chrom enthält, jeweils bezogen auf das Gesamtgewicht des Hydrierkatalysators und gegebenenfalls Füllstoffe mit Rest auf 100 Gew.-%.

11. Verfahren gemäß einem oder mehreren der Ansprüche 1 bis 10, **dadurch gekennzeichnet, dass** die umzusetzenden aliphatischen Aldehyde als Gemisch von aliphatischen Aldehyden mit unterschiedlicher Kohlenstoffzahl verwendet werden.

12. Verfahren gemäß einem oder mehreren der Ansprüche 1 bis 11, **dadurch gekennzeichnet, dass** ein Gemisch aus geradkettigen n- und verzweigtkettigen iso-Aldehyden, die 13 und 15 Kohlenstoffatome im Molekül enthalten, verwendet werden.

## Claims

1. Continuous process for preparing primary aliphatic amines having 3 to 18 carbon atoms by reacting corresponding aliphatic aldehydes with ammonia and hydrogen in the presence of a hydrogenation catalyst, **characterized in that** the reaction is carried out without solvent at a molar ratio of aliphatic aldehyde to ammonia of more than 1 to 16, above the critical temperature and above the critical pressure of ammonia.

2. Process according to Claim 1, **characterized in that** the molar ratio of aliphatic aldehyde to ammonia is 1 to at least 18, preferably 1 to at least 20.

3. Process according to Claim 1 or 2, **characterized in that** the molar ratio of aliphatic aldehyde to ammonia is from 1 to 18 to 1 to 60, particularly from 1 to 20 to 1 to 50.

4. Process according to one or more of Claims 1 to 3, **characterized in that** the reaction is conducted at a temperature above 132.5°C to 190°C, particularly above 132.5°C to 180°C and especially preferably above 160°C to 180°C.

5. Process according to one or more of Claims 1 to 4, **characterized in that** the reaction is conducted at a pressure above 11.4 to 20 MPa, particularly above 12.8 to 20 MPa and especially preferably 13 to 18 MPa.

6. Process according to one or more of Claims 1 to 5, **characterized in that** the aliphatic aldehydes to be reacted contain 5 to 15, preferably 8 to 15, carbon atoms in the molecule.

7. Process according to one or more of Claims 1 to 6, **characterized in that** the hydrogenation catalyst contains at least nickel, cobalt, platinum, palladium, iron, rhodium or copper.

8. Process according to one or more of Claims 1 to 7, **characterized in that** the hydrogenation catalyst contains a support material.

9. Process according to one or more of Claims 1 to 8, **characterized in that** the hydrogenation catalyst contains oxides of calcium, of barium, of zinc, of aluminum, of zirconium, of chromium or mixtures thereof, as additives.

10. Process according to one or more of Claims 1 to 9, **characterized in that** the hydrogenation catalyst used is a nickel catalyst containing 20% to 60% by weight of nickel, from 20% to 70% by weight of kieselguhr and from 10% to 20% by weight of chromium, in each case based on the total weight of the hydrogenation catalyst and optionally fillers making up the balance to 100% by weight.

11. Process according to one or more of Claims 1 to 10, **characterized in that** the aliphatic aldehydes to be reacted are used as a mixture of aliphatic aldehydes having different numbers of carbon atoms.

12. Process according to one or more of Claims 1 to 11, **characterized in that** a mixture of straight-chain n- and branched-chain iso-aldehydes containing 13 and 15 carbon atoms in the molecule is used.

## Revendications

1. Procédé continu de fabrication d'amines aliphatiques primaires de 3 à 18 atomes de carbone par mise en réaction d'aldéhydes aliphatiques correspondants avec de l'ammoniac et de l'hydrogène en présence d'un catalyseur d'hydrogénation, **caractérisé en ce que** la réaction est réalisée sans solvant à un rapport molaire entre l'aldéhyde aliphatique et l'ammoniac de plus de 1 sur 16 au-dessus de la température critique et au-dessus de la pression critique de l'ammoniac.

2. Procédé selon la revendication 1, **caractérisé en ce que** le rapport molaire entre l'aldéhyde aliphatique et l'ammoniac est de 1 sur au moins 18, de préférence de 1 sur au moins 20.

3. Procédé selon la revendication 1 ou 2, **caractérisé en ce que** le rapport molaire entre l'aldéhyde aliphatique et l'ammoniac est de 1 sur 18 à 1 sur 60, notamment de 1 sur 20 à 1 sur 50.

4. Procédé selon une ou plusieurs des revendications 1 à 3, **caractérisé en ce que** la réaction a lieu à une température de plus de 132,5 °C à 190 °C, notamment de plus de 132,5 °C à 180 °C et de manière tout particulièrement préférée de plus de 160 °C à 180 °C.

5. Procédé selon une ou plusieurs des revendications 1 à 4, **caractérisé en ce que** la réaction a lieu à une pression de plus de 11,4 à 20 MPa, notamment de plus de 12,8 à 20 MPa, et de manière tout particulièrement préférée de 13 à 18 MPA.

6. Procédé selon une ou plusieurs des revendications 1 à 5, **caractérisé en ce que** les aldéhydes aliphatiques mis en réaction contiennent 5 à 15, de préférence 8 à 15, atomes de carbone par molécule.

7. Procédé selon une ou plusieurs des revendications 1 à 6, **caractérisé en ce que** le catalyseur d'hydrogénation contient au moins du nickel, du cobalt, du platine, du palladium, du fer, du rhodium ou du cuivre.

8. Procédé selon une ou plusieurs des revendications 1 à 7, **caractérisé en ce que** le catalyseur d'hydrogénation contient un matériau support.

9. Procédé selon une ou plusieurs des revendications 1 à 8, **caractérisé en ce que** le catalyseur d'hydrogénation contient en tant qu'additifs des oxydes de calcium, de baryum, de zinc, d'aluminium, de zirconium, de chrome ou leurs mélanges.

10. Procédé selon une ou plusieurs des revendications 1 à 9, **caractérisé en ce qu'**un catalyseur de nickel qui contient 20 à 60 % en poids de nickel, 20 à 70 % en poids de diatomée et 10 à 20 % en poids de chrome est utilisé en tant que catalyseur d'hydrogénation, à chaque fois par rapport au poids total du catalyseur d'hydrogénation, le reste jusqu'à 100 % en poids étant éventuellement des charges.

11. Procédé selon une ou plusieurs des revendications 1 à 10, **caractérisé en ce que** les aldéhydes aliphatiques mis en réaction sont utilisés sous la forme d'un mélange d'aldéhydes aliphatiques ayant un nombre de carbones différent.

12. Procédé selon une ou plusieurs des revendications 1 à 11, **caractérisé en ce qu'**un mélange de n-aldéhydes linéaires et d'iso-aldéhydes ramifiés, qui contiennent 13 et 15 atomes de carbone par molécule, est utilisé.
